# EUROPEAN PATENT APPLICATION

(11) **EP 2 803 318 A1**
(43) Date of publication of application: **19.11.2014**
(21) Application number: 14168438.1
(22) Date of filing: 15.05.2014
(51) Int. Cl.: A61B 5/055, G01R 33/28

(54) **Control of SAR values in MR imaging**

(30) Priority: 17.05.2013 US 201361824454 P
(71) Applicant: Imris Inc., Winnipeg, Manitoba R3T 1N5 (CA)
(72) Inventor: Zhu, Haoqin, Winnipeg, Manitoba R3T 1N5 (CA); Schellekens, Wayne, Winnipeg, Manitoba R3T 1N5 (CA); Petropoulos, Labros, Winnipeg, Manitoba R3T 1N5 (CA)
(74) Representative: Jostarndt, Hans-Dieter

(57) **Abstract**

In a method for imaging a body part of a patient particularly in relation to DBS where conductive elements can reduce a safe SAR level, the power in the RF pulses being delivered to the RF transmit coil is measured in real time by a unit associated with the FR transmit coil and is used to stop the supply of RF pulses to the RF coil in the event that the power exceeds a predetermined safe limit. As signal can also be transmitted to the MR control unit to shut off the imaging sequence.

## Description

This invention relates to a method of imaging while controlling maximum SAR values to avoid damage to the patient by application of excess RF power.

### BACKGROUND OF THE INVENTION

It is conventional in MR imaging that the scanner control system ensures safety of the patient with respect to RF energy absorbed by the patient by calculating and controlling a specific-absorption rate (SAR) setting. The SAR setting is based on many parameters including the patient height, weight, the anatomy to be imaged and the position of the patient table in the bore.

The introduction of Deep Brain Stimulation (DBS) leads and other conductive structures under guidance by Magnetic Resonance Imaging is a recent development in clinical procedure and has led to difficulties in SAR control since the presence of the conductor in the imaging zone dramatically increases the effect of the RF signals on the part of the patient concerned. Various papers have been written characterizing the MR environment and analyzing how the heat is generated by the RF field applied during the MRI for implanted devices. Significant efforts have attempted to modify the resonant structure of the implant, to detect when energy is being coupled into an implanted device or otherwise to detect unsafe conditions.

Thus during MR scanning, it is well known that the high intensity electric fields created around the tips of long conductive structures inside a patient's body will create high current densities in the tissue and thus increased temperatures that could burn the tissue. It can however be difficult to predict when hazardous temperatures will be created because of the variability of the electrical properties of the patient's tissue, the variability of the geometry of the antenna structures involved, the variability of the MR systems that are used and the variability of the associated equipment that is used to implant the device.

It has been well established that DBS leads require a very low specific absorption rate (SAR) level (0.1 W/kg) for safe scanning when DBS leads are present. Common MR scanners only use the federal mandated SAR levels of 2W/kg and 4W/kg.

When a patient is placed beneath a MR coil, it 'loads' the coil which causes detuning, a drop in Q factor (lost efficiency) and creates a mismatch between the system and coil. As it is desirable to use the coils for multiple patient types and a variety of positions and body parts, the load as seen by the system can vary greatly.

According to one recommendation, generating imaging sequences based on SAR is problematic as the SAR is a calculated estimate, not a measured value.

The protocols are normally adjusted to achieve optimum images, however due to patient loading, the optimum adjustment may exceed the safe delivered SAR limit. In order to ensure safe limits, the operator must de-rate the parameters to ensure safe operation based on the worst case load.

US Patent 5,730,134 (Dumoulin) issued March 24 1998 to General Electric discloses the use of a system in which automatically an MRI scan is terminated or reduced in power in response to detection of a raised temperature within the body of the patient caused by RF heating of an electrode within the patient. The electrode is located within an invasive device inserted into the body and the temperature sensor is an optical thermocouple mounted also within the invasive device.

US Patent 5,209,233 (Holland) issued May 11 1993 to Picker International discloses a similar system in which automatically an MRI scan is terminated in response to detection of a raised temperature at a cardiac monitoring electrode attached to the patient to prevent burning. The temperature sensor is an optical thermocouple mounted within the clip.

US Patent 6,185,443 (Crowley) issued February 6 2001 to Boston Scientific discloses an interventional device for minimally invasive diagnostic and therapeutic procedures where the device or probe carries sensors with a display on a distal end of the device to display to the user the sensed data including temperature.

### SUMMARY OF THE INVENTION

It is one object of the invention to provide a method of imaging which ensures that SAR limits are not exceeded.

According to the invention there is provided a method for imaging a body part of a patient comprising:
operating an MR magnet with gradient coil to generate a variable magnetic field to be applied to the patient;
operating an RF transmit coil arrangement for supplying RF pulses to an RF transmit coil in a transmit stage to be applied through the RF transmit coil to the patient to be imaged such that the patient generates an MR signal in response to the magnetic field and the RF signal applied; acquiring the MR signal in a receive stage; processing the MR signal by which an image is generated; measuring the power in the RF pulses being delivered to the RF transmit coil in real time; and stopping the supply of RF pulses to the RF coil in the event that the power exceeds a predetermined safe limit.

Preferably the measuring step is arranged using parameters related to the patient weight etc to measure SAR (Watts/kg) being delivered to the patient in real time.

Preferably the stopping of the RF pulses acts to ensure the safety of a transmit coil which is independent of patient loading and use.

In one arrangement the RF pulses are stopped by supplying a signal directly to an MR imaging system controlling the RF transmit coil arrangement to stop the imaging sequence. In this arrangement, in addition for a fail-safe operation, the RF pulses are also stopped by disconnecting RF coil.

In another arrangement, particularly where the MR imaging system has no input allowing halting of the imaging sequence, the RF pulses are stopped simply by disconnecting the RF transmit coil.

Preferably the safe limit is the SAR value which is determined including parameters of the patient since SAR values are well known and previously calculated. However it is the power value which is actually determinative of the potential damage to the patient.

Preferably the power is measured at a measurement unit associated with the RF transmit coil and separate from the MR imaging system. However the imaging system may include a measuring system which measures the actual power being supplied as opposed to the conventional technique which calculates the pulse sequence using predetermined parameters in order to provide a sequence which is predicted or calculated to generate a required SAR value.

Preferably the parameters of the patient are input manually into the measurement unit so that the unit has an interface for a manual input by the operator. As an alternative, the parameters can be obtained from the MR imaging system where of course the parameters concerned must be input before the imaging sequence is calculated.

Preferably the power is measured by continually measuring instantaneous transmitted power which is integrated to calculate the average power delivered to the patient. When that average power exceeds the predetermined maximum value, the system shuts down the supply of the pulses to the RF transmit coil to ensure no damage can occur.

Preferably the power is measured at a controller which contains a microprocessor and driver circuitry to turn off an RF Switch to the RF transmit coil.

The power can be measured by detecting transmitted power through a directional coupler in a lead to the RF transmit coil. In addition or alternatively a sampling coil can be located at the RF transmit coil and is used as an alternative to or in addition to the directional coupler to detect the actual RF power applied.

Also a bi-directional coupler can be used where the power is measured also by detecting reflected power signal from the bi-directional coupler in a lead to the RF transmit coil. In this way the power can be measured using one, two or all of the three separate techniques. This can ensure that errors or failures in any one of the systems can be found as a duplicative system to provide a fail-safe action.

The operator is therefore not restricted and can obtain better images if the SAR being delivered is measured using the RF transmit coil itself. In this manner, the operator can achieve maximum quality without exceeding safe limits. The operator therefore is not restricted to predetermined maximum calculated values and can err on the side of obtaining the best images by increasing the power supplied, safe in the knowledge that the actual power supplied cannot exceed the maximum value.

This invention measures the SAR (Watts / kg) being delivered to the patient in real time, and will stop the scan if the safe limit is exceeded. It does this by measuring the actual power delivered to the patient using directional couplers in the RF transmit coil. When the safe power level is exceeded a RF switch opens up to prevent any more RF from reaching the patient, and a logic signal can be sent to stop the scan.

For scanners that do not have a logic control input to stop the scan, the opening of the RF switch causes high reflected power that will force the scanner to terminate the sequence.

To calculate SAR properly, the system requires parameters including the patient weight and age. The size or height of the patient is not a large contributor to the head size, which is necessary for calculating the SAR relative to the specific body part to be imaged. This can be manually entered into the controller before scanning starts, or can be automatically extracted from the scanner console before scanning commences. The patient weight is scaled according to the body part being exposed to the RF field, using the coil size and human body model.

The invention has been described in the context of DBS lead implantation but it is also relevant to and can be used with the introduction of any invasive conducting structure inserted within the body during MR imaging and is not limited to imaging of the head. This could also be for interventional applications where catheters are placed in blood vessels.

### BRIEF DESCRIPTION OF THE DRAWINGS

One embodiment of the invention will now be described in conjunction with the accompanying drawings in which:

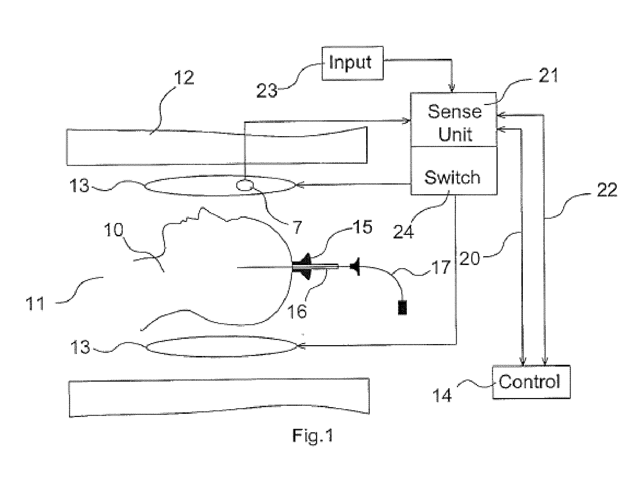

Figure 1 is a schematic illustration of an MR imaging system used for insertion of a DBS lead into the brain of a patient.
Figure 2 is a schematic of the SAR/power control unit of Figure 1.
Figure 3 is a schematic of the SAR/power control unit of Figure 1 using a bi-directional coupler to allow measurement of reflected power where the sample coil 7 is omitted.

In the drawings like characters of reference indicate corresponding parts in the different figures.

### DETAILED DESCRIPTION

The apparatus of the present invention is mainly shown only schematically as many of the components are well known to persons skilled in this art. In particular there is shown a part of the patient shown at 10 which is located during at least a part of the procedure in an MR imaging system 11 including a magnet 12, RF coils 13 and a control and display system 14 for use in an invasive procedure for insertion of a conductive device into a patient while the insertion is guided using Magnetic Resonance Imaging. The conventional components further include a stereotactic positioner 15 and a cannula 16 located by the positioner 15 so as to define a path for insertion of a conductive device to a target location.

The RF pulses generated at the MR system 14 are transmitted to the RF coil 13 along a conductor 20 which supplies those signals to a sensing unit 21 forming part of the coils 13 or associated with the coils 13. The sensing unit 21 includes an RF switch 24. The unit 21 can communicate to and from the control unit 14 along a communication path 22.

The conventional components further include a conductive electrode 17 which in regard to DBS can be either a micro-recording electrode that is temporarily positioned at the target or a stimulation electrode intended to be located at and remain in position at a target location in the brain of the patient.

In the method for embedding a conductive device into a patient the following steps are applied:
the stereotactic positioner is attached to the body of the patient and adjusted until its trajectory guide is oriented along the desired trajectory;
the sheath with a stylet inserted therein is fed through the trajectory guide along the desired trajectory until it reaches the target location, using Magnetic Resonance Imaging of the target location and the trajectory;
the stylet is removed and replaced with the electrode;
during the MRI, data relating to the power of the RF signals supplied is detected at the sensing unit 21;
when imaging has confirmed that the conductive element is indeed located at the target location, the conductive element is fixed in place and the sheath is removed leaving the conductor in place.

The imaging setup sequence is as follows:

The coil 13 is plugged into the scanner control 14 and the patient weight is entered into the unit 21 by a manual interface 23 or read from the scanner console 14. The switch 24 is set to allow the transmitted RF to reach the coil 13.

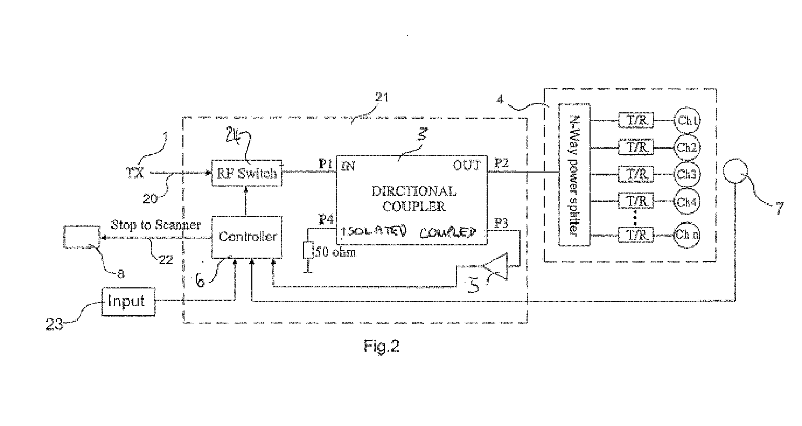

Turning now to Figure 2, a directional coupler 3 samples the transmitted power and through detector 5, passes it on to an integrator/controller 6. The function of the integrator/controller is to sum the applied power to calculate the average SAR delivered to the patient.

The controller 6 contains a microprocessor and driver circuitry to turn off the RF Switch 24 when the calculated SAR exceeds the safe limit that is programmed into the unit (for example, 0.1 W/kg for DBS leads).

If the scanner does not receive the stop signal 8 on the conductor 22, the high reflected power from the open switch 24 will cause the current sequence to abort at the controller 14. For use of a volume coil as the RF transmit coil 13, a sampling coil 7 located at the RF transmit coil 13 can be used in place of, or along with the coupled supply signal to detect the actual RF power applied and to stop the scanner.

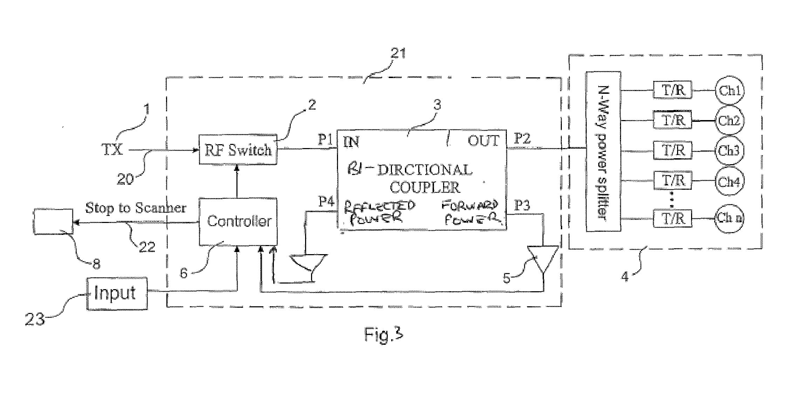

Another method shown in Figure 3 of detecting the amount of SAR or power being delivered is to utilize the reflected power signal from a bi-directional coupler 3. The ports are labelled 'reflected power' and 'forward power'. The use of both detected values of the forward power and the reflected power has the advantage of providing an indication of the current loading and efficiency of the transmit coil, which is highly dependent on the patient loading and coil match.

This arrangement has the benefit of forcing the scan to be stopped if the operator attempts to exceed safe limits for imaging when a patient with implants is being imaged.

The SAR Calculation is typically based on a human cylinder model including the head, torso and two leg cylinders. This cylinder model is calculated for each patient depending on registration data (age, size, weight). According to the coil length it is assumed that the corresponding cylinder part (length and mass) is exposed to the B1-field. With these data, a calculation is made to set the B1 power limit that delivers the maximum SAR value of 0.1W/kg. This is compared with the measured value and if the measured value is higher, the scanning is stopped using the methods described earlier.

## Claims

1. A method for imaging a body part of a patient comprising:
operating an MR magnet with gradient coil of an MR imaging system to generate a variable magnetic field to be applied to the patient;
operating an RF transmit coil arrangement of an MR imaging system for supplying RF pulses to an RF transmit coil in a transmit stage to be applied through the RF transmit coil to the patient to be imaged such that the patient generates an MR signal in response to the magnetic field and the RF signal applied;
acquiring the MR signal in a receive stage;
processing the MR signal by which an image is generated;
measuring the power in the RF pulses being delivered to the RF transmit coil in real time;
and stopping the supply of RF pulses to the RF coil in the event that the power exceeds a predetermined safe limit.

2. The method according to claim 1 wherein SAR (Watts/kg) being delivered to the patient in real time is measured.

3. The method according to any preceding claim wherein the stopping of the RF pulses acts to ensure the safety of a transmit coil which is independent of patient loading and use.

4. The method according to any preceding claim wherein the RF pulses are stopped by disconnecting the RF coil.

5. The method according to any preceding claim wherein the RF pulses are stopped by supplying a signal to the MR imaging system.

6. The method according to any preceding claim wherein the safe limit is determined including parameters of the patient.

7. The method according to any preceding claim wherein the power is measured at a measurement unit associated with the RF transmit coil and separate from the MR imaging system.

8. The method according to claim 7 wherein the safe limit is determined including parameters of the patient and the parameters are input manually into the measurement unit.

9. The method according to claim 7 wherein the safe limit is determined including parameters of the patient and the parameters are obtained from the MR imaging system.

10. The method according to any preceding claim wherein the power is measured by continually measuring instantaneous transmitted power which is integrated to calculate the average power delivered to the patient.

11. The method according to any preceding claim wherein the power is measured by detecting transmitted power through a directional coupler in a lead to the RF transmit coil.

12. The method according to claim 11 wherein a sampling coil is located at the RF transmit coil and is used in addition to the directional coupler to detect the actual RF power applied.

13. The method according to any preceding claim wherein the power is measured also by detecting reflected power signal from a bi-directional coupler in a lead to the RF transmit coil.
